# EUROPEAN PATENT APPLICATION

(11) **EP 4 534 997 A1**
(43) Date of publication of application: **09.04.2025**
(21) Application number: 23814613.8
(22) Date of filing: 26.05.2023
(51) Int. Cl.: G01N 33/00, G01N 33/14

(54) **METHOD FOR QUANTIFYING THE ASTRINGENCY OF BEVERAGES OR FOOD BY MEANS OF PROTEINS THAT ARE REACTIVE WITH TANNINS**

(30) Priority: 02.06.2022 CL 20221467
(71) Applicant: Pontificia Universidad Católica De Chile, 8331150 Santiago (CL); Universidad de Santiago de Chile, Santiago (CL)
(72) Inventor: BROSSARD ARAVENA, Natalia Daniela, Santiago (CL); O'BRIEN OPAZO, José Antonio, Santiago (CL); BORDEU SCHWARZE, Edmundo, Santiago (CL); OSORIO LIRA, Fernando Alberto, Santiago (CL)
(74) Representative: Elzaburu S.L.P.
(86) International application number: PCT/CL2023/050044
(87) International publication number: WO 2023/230737

(57) **Abstract**

The invention provides a method for quantifying the astringency of beverages or food based on a composition comprising proteins that are reactive with tannins. The method determines the astringency by means of oral tribology, wherein the proteins used are similar to those of human saliva and are characterized in that they are highly interaction with tannins.

The invention further relates to a kit provided to quantify sensory astringency in food or beverages, preferably in fermented alcoholic beverages, by means of the method of the present invention.

## Description

### FIELD OF THE INVENTION

The invention is part of the food and agricultural industry. In particular, the invention focuses on a method and kit for determining the astringency of food products, preferably fermented alcoholic beverages.

The invention provides a method and kit for determining astringency for use in quality control and applications related to the manufacture of food and beverages.

### BACKGROUND

The sensation of dryness and roughness in the mouth after tasting a wine is called astringency and is mainly given by the amount of tannins and polyphenols. This oral perception is one of the main quality factors in many foods such as red wines, teas, and fruits. There are several hypotheses that try to explain the mechanisms by which astringency is perceived. However, it is still unknown which of them prevails. Most authors agree that astringency is a tactile stimulus (physical perception), which arises from the precipitation of saliva proteins with wine tannins, causing a loss of lubrication in the mouth.

In the food industry, astringency is measured at various stages of the beverage and food production process.

A first challenge facing the evaluation of astringency in wines is the quantification methodology. For example, in the case of red wine, each measurement is carried out through trained panels and/or oenologists, which implies a high cost in man hours, both in the training stage for the perception of this parameter and in each of the production phases in which the analysis itself is carried out. Despite the training, sensory evaluation always retains a degree of subjectivity and variability in the results obtained, which also vary depending on the participating judges or the different evaluation sessions carried out. In addition, as it is a perception that prevents oral lubrication, it is a very exhausting activity for tasters or winemakers.

There are some approaches to the measurement of sensory astringency by means of complementary chemical analyses based on the determination of tannins and concentrations, but a correlation between the astringency of the wine and the amount or type of tannins in the wine has not been demonstrated. Therefore, the results of this type of measurement are not reliable for oenologists and winemakers.

Among the analytical methods used to measure astringency are colorimetric methods that use a color development reaction, measurement of multiple wavelengths, precipitation of tannins by non-protein materials (methylcellulose), and precipitation of tannins by proteins (gelatin, ovalalbumin, BSA, and turbidity). Among the physical measurements used are rheological measurements that relate viscosity to the body of the wine.

### PREVIOUS ART

Various methods are known in the industry that address the problem of measuring sensory astringency, particularly in wines.

On the one hand, in the publication by Brossard et al. (2016), by the same inventors, the sensory perception of astringency is analyzed using tribology techniques using saliva in contact with the compositions to be evaluated and compared with human perception.

The publication Laguna, L., et al (2017) analyzes the perception in the mouth, of wines, by a panel of experts and uses instrumental methods in which tribology is used. Human saliva (HS) is used in this document for the analyses and does not suggest the use of transgenic proteins or artificial saliva.

In the document by Laguna, L., & Sarkar (2017), a review of the use of tribology to measure astringency in wines is made and indicates that artificial saliva has been used, where it is suggested to use artificial saliva that contains at least mucin.

On the other hand, in Yeom, J. et al (2020) a hydrogel containing mucin and electrolytes is used and covering an artificial tongue, where the system detects the formation of protein aggregates in response to astringent compounds due to changes in the conductivity of the hydrogel.

Finally, in the publication CN107764762 A (2018), artificial saliva with salivary amylase is used to evaluate astringency, although the objective points to the influence of tobacco smoke on the astringency of the oral cavity.

In summary, in previous art there are several approaches to determine the astringency of a beverage or food, however, not all of them have optimized a uniform method that uses a standardized composition to measure the sensory astringency of beverages, particularly fermented beverages with or without alcohol.

### BRIEF DESCRIPTION OF THE FIGURES

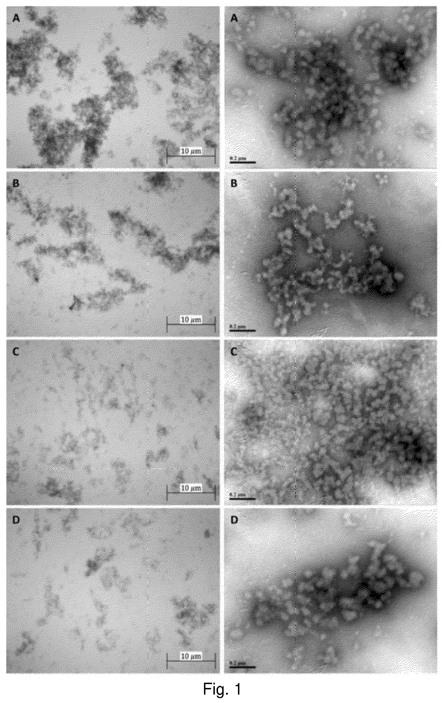

Figure 1. Micrographs of wine and saliva mixtures in a 1:1 ratio. The first column shows micrographs using a light microscope and the second column corresponds to electron transmission micrographs: a) Cabernet sauvignon Press., b) Cabernet sauvignon, c) Carménère, d) Merlot.
Figure 2. Comparison of friction coefficients between samples with addition of different levels of skin and seed tannin over a wide range of travel rates using artificial saliva with a mixture of Protein 1 (alpha-amylase).
Figure 3.1 Astringency and friction coefficient ratio in wines alone. 3.2 Tannic drying ratio and coefficient of friction in wines alone. 3.3 Coefficient of friction and astringency, mixture with artificial saliva. 3.4 Coefficient of friction and dryness, mixture with artificial saliva.

### DESCRIPTION OF THE INVENTION

The invention provides an objective, optimized and highly accurate method for quantifying sensory astringency in food products, based on tannin-reactive proteins.

The method is based on the determination of astringency by oral tribology using an artificial saliva-type protein composition (protein composition or artificial saliva).

The invention also provides a kit for quantifying astringency by the method of the present invention in foods or beverages, preferably fermented alcoholic beverages.

During the development of the invention, tests were carried out on a sample of wines with different astringencies, in a wide variability of values, and the results were validated with sensory tests. Initially, mixtures of human saliva were worked with, and later a protein composition or artificial saliva was developed, with specialized proteins that replicate the interactions that give rise to the sensory perception of astringency. The use of artificial saliva is more practical, it reduces costs, standardizes measurement and optimizes the parameters evaluated.

The selected protein corresponds to the one that presents a greater interaction with tannins and polyphenols and that allows a correlation with sensory astringency to be obtained. A protein with the same reactivity as saliva proteins is selected.

In a preferential embodiment of the invention, artificial saliva proteins are reactive to the tannins in the wine.

In a preferential embodiment of the invention, the artificial saliva protein corresponds to a mixture comprising the enzyme alpha-amylase □□-amylase) and optionally includes the addition of at least one protein selected from mucins, amylases, proline-rich proteins and lysozymes.

The concentration of proteins in artificial saliva is in the range of 0.5 to 2.5 mg/L, preferably 0.75 to 1.75 mg/L.

### Method

Preliminarily, an astringency scale with extreme values was performed to calibrate the astringency measurement method.

To validate and contrast the instrumental results obtained, the same wines characterized in their analytical skeleton were sensorily validated.

The method comprises the steps of:
- Providing an artificial saliva-type protein composition; where the protein corresponds preferably to alpha-amylase and optionally includes at least one protein selected from mucins, amylases, proline-rich proteins and lysozymes, dissolved in ultrapure or distilled water.
- Contacting the protein composition with the drink; where the protein composition is mixed in a ratio between 0.75:2 to 1.75:0.5, preferably between 0.75:1.5 to 1.25:1, and more preferably in a ratio of 1:1.
- Determining the tribological profile using a tribological device, obtaining the analysis of the coefficient of friction; and
- Determining the astringency of the drink.

The concentration of proteins in artificial saliva is in the range of 0.5 to 2.5 mg/L, preferably between 0.75 to 1.75 mg/L, preferably between 0.75 to 1.5 mg/L, and more preferably 1 mg/L.

The coefficient of friction is analyzed by subjecting the sample to a normal force of 1N to 5N and at a temperature in a range of 25 to 30ºC, preferably at a temperature of 28±1ºC. The analysis is performed by subjecting the sample to a normal force for between 10-40 min, preferably between 25 to 30 min.

The measurement is made with a ratio between the turning movement and the sliding of the ball on the disc *(Sliding Rotation Ratio, SRR)* of 25 to 200% and a speed between 1 to 20mm/S, preferably between 5 to 10mm/S.

### Kit

The invention also provides a product or kit for the measurement of sensory astringency in red wines. The kit comprises;
a) an artificial saliva-type protein composition containing alpha-amylase and optionally includes at least one protein selected from mucins, amylases, proline-rich proteins and lysozymes where the protein concentration in the solution is between 0.5 to 2.5 mg/L;
b) a tribological device or specific adapter for measuring astringency comprising a tribological pair consisting of a polydimethylsiloxane disc (PDMS), PDMS ball preferably both of hardness index 30 or 50 (HARD 30 or 50) and the ball preferably of a size of 1/2" to ¾".

Although the scope of the invention is primarily oriented to its application in the wine industry, this should not be understood as a limitation of the invention, since methods and kits according to the invention could be applied in any industry that produces food or beverages rich in polyphenols.

The technology could be applied, for example, in fruit or plant juices or extracts, fermented beverages (such as beer, cider, or wine), or distilled alcoholic beverages, among others.

### EXAMPLES

Examples of the realization of the invention are described below by way of illustration, without limiting the technical variants that may be incorporated or modified by a person skilled in the art, and which are within the scope of the present invention.

### Example 1: Preliminary Evaluation of the Method

The MTM2 tribometer (Mini Traction Machine 2, PCS Instruments) is used under a *Sliding Rotation Ratio* (SRR) condition of 50% and analysis of the friction coefficient obtained under conditions of fixed speed at 5mm/S and 1 Newton of load for 30 minutes. The SRR or Sliding Rolling ratio, is the quotient between the turning movement and the sliding of the ball on the disc.

Evaluations are performed mixed in a 1:1 ratio with artificial saliva. The artificial saliva was preliminarily prepared with 1 mg of α-amylase (of fungal origin) in 1 mL of MiliQ water, i.e. 1 g/L and 1-2 minutes of stirring.

The disk and ball of hard PDMS 50 and the ball of a size of 3/4" were selected.

### Example 2: Comparison

To assess the protein composition in artificial saliva, a study was conducted using a combination of two commercial salivary proteins, alpha-amylase and mucin in different concentrations. The measurements were made using a white wine solution with the addition of skin tannins and seeds to simulate perceptions of astringency and dryness, respectively. The concentrations used of each of the tannins mentioned above are listed in Table 1. It should be noted that the tannin concentrations used in this study are the same as those used for the training of the sensory panel in the parameters of astringency and dryness.

**Table 1. Tannin concentrations used to optimize the amount of proteins present in the artificial saliva solution.**

| | **Astringency** | **Dryness** |
|---|---|---|
| | Skin tannin (g/L) | Seed tannin (g/L) |
| Level 1 | 0.3 | 0.2 |
| Level 2 | 0.6 | 0.4 |
| Level 3 | 1.2 | 0.8 |
| Level 4 | 2.4 | 1.6 |

The trials were carried out with mixtures called Protein 1 (alpha-amylase 1 mg/L) and Protein 2 (alpha-amylase 0.8 mg/L; mucin 0.2 mg/mL). Both artificial saliva solutions were tested against each of the tannin concentrations listed in Table 1 in a 1:1 ratio. Tribological coefficient of friction measurements were performed using an MTM-2 (Mini Traction Machine) from PCS Instruments.

The operation of the equipment is summarized as follows. A small amount of sample is fed into the system in the steel container. A ball exerts a normal force against the face of the disc and both samples move independently, both ball and disc components are called tribological pairs. Both components can be made of different materials, among the most frequently used are steel, rubber and the polymer poly-dimethylsiloxane also known as PDMS. The frictional force between the two samples is automatically measured and recorded throughout the test.

For this experiment, both the hard PDMS 50 disc and ball and the 3/4" ball were used. The samples of artificial saliva and model wine were analyzed in a ratio of 1:1 at a temperature of 28ºC, with a normal force of 1N, an SRR *(Sliding Rotation Ratio)* value of 50% and in a range of travel speeds from 1 to 20 mm/s.

### Analysis Protein 1 (alpha-amylase 1 mg/mL)

**Table 2. Friction coefficients in a range of travel speeds from 1 to 8 mm/s of model wine with different doses of skin tannin versus artificial saliva with Protein 1 (alpha-amylase)**

| | **V1** | **V2** | **V3** | **V4** | **V5** | V6 | **V7** | V8 |
|---|---|---|---|---|---|---|---|---|
| Astringency 1 | 0.483 a* | 0.472 a | 0.464 a | 0.447 a | 0.446 a | 0.416 a | 0.427 a | 0.431 a |
| Astringency 2 | 0.452 a | 0.368 b | 0.412 a | 0.335 b | 0.318 b | 0.290 b | 0.306 b | 0.276 b |
| Astringency 3 | 0.200 b | 0.253 c | 0.309 b | 0.308 b | 0.309 b | 0.257 b | 0.254 bc | 0.220 bc |
| Astringency 4 | 0.167 b | 0.176 c | 0.260 b | 0.280 b | 0.290 b | 0.248 b | 0.220 c | 0.169 c |
| Prob > F | 0.000 | <0.0001 | 0.000 | 0.001 | 0.000 | 0.001 | 0.000 | <0.0001 |
| Significance | Yes | Yes | Yes | Yes | Yes | Yes | Yes | Yes |

| | | | | | | | | |
|---|---|---|---|---|---|---|---|---|
| *Values followed by the same letter in a column are not different (P: 0.05) | | | | | | | | |

**Table 3. Friction coefficients over a range of travel speeds from 1 to 8 mm/s of model wine with different doses of seed tannin versus artificial saliva with Protein 1 (alpha-amylase)**

| | **V1** | **V2** | **V3** | **V4** | **V5** | V6 | **V7** | V8 |
|---|---|---|---|---|---|---|---|---|
| Dryness 1 | 0.560 a* | 0.488 a | 0.535 a | 0.493 a | 0.480 a | 0.432 a | 0.397 a | 0.384 a |
| Dryness 2 | 0.381 ab | 0.362 b | 0.406 ab | 0.344 b | 0.343 b | 0.306 b | 0.331 a | 0.284 b |
| Dryness 3 | 0.317 b | 0.262 c | 0.330 b | 0.266 bc | 0.219 c | 0.193 c | 0.196 b | 0.168 c |
| Dryness 4 | 0.223 b | 0.189 c | 0.294 b | 0.246 c | 0.213 c | 0.213 bc | 0.196 b | 0.159 c |
| Prob > F | 0.004 | <0.0001 | 0.004 | <0.0001 | <0.0001 | 0.000 | <0.0001 | 0.000 |
| Significance | Yes | Yes | Yes | Yes | Yes | Yes | Yes | Yes |

| | | | | | | | | |
|---|---|---|---|---|---|---|---|---|
| *Values followed by the same letter in a column are not different (P: 0.05) | | | | | | | | |

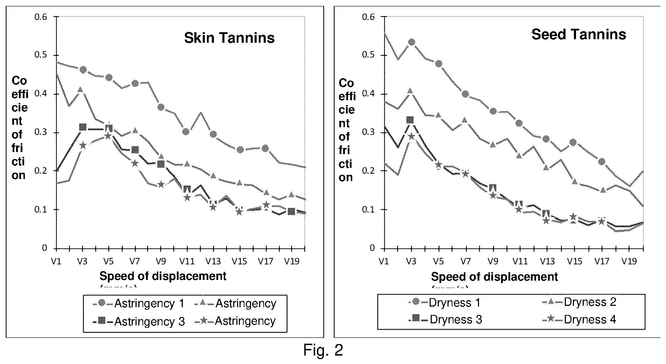

According to the results obtained after measuring artificial saliva containing protein 1 (alpha-amylase, 1 mg/mL) and wine with the addition of skin tannins (Table 2) and seed tannin (Table 3), it is observed that by means of an analysis of variance performed on the friction coefficients obtained, it is possible to differentiate samples of different levels of astringency and dryness at the same speed of displacement. Additionally, at low speeds, the difference between samples of different levels of astringency and dryness is much better, with the speed of 2 (mm/s) being the best to differentiate both parameters in model wine. The above is best seen graphically in Figure 2.

### Protein 2 analysis (alpha-amylase 0.8 mg/mL and mucin 0.2 mg/mL)

**Table 4. Coefficients of friction over a range of travel velocities from 1 to 8 mm/s of model wine with different levels of skin tannin versus artificial saliva with Protein 2 (alpha-amylase plus mucin)**

| | **V1** | **V2** | **V3** | **V4** | **V5** | V6 | **V7** | **V8** |
|---|---|---|---|---|---|---|---|---|
| Astringency 1 | 0.683 a* | 0.894 ab | 0.712 a | 0.571 a | 0.494 a | 0.414 a | 0.391 a | 0.331 a |
| Astringency 3 | 0.424 a | 0.462 b | 0.404 b | 0.317 a | 0.395 a | 0.278 a | 0.471 a | 0.395 a |
| Astringency 2 | 0.494 a | 0.943 a | 0.722 a | 0.608 a | 0.554 a | 0.413 a | 0.438 a | 0.304 a |
| Astringency 4 | 0.449 a | 0.484 ab | 0.358 b | 0.351 a | 0.345 a | 0.278 a | 0.402 a | 0.322 a |
| Prob > F | 0.301 | 0.016 | 0.002 | 0.076 | 0.172 | 0.474 | 0.855 | 0.336 |
| Significance | No | Yes | Yes | No | No | No | No | No |

| | | | | | | | | |
|---|---|---|---|---|---|---|---|---|
| *Values followed by the same letter in a column are not different (P: 0.05) | | | | | | | | |

**Table 5. Coefficients of friction over a range of travel speeds from 1 to 8 mm/s of model wine with different levels of seed tannin versus artificial saliva with Protein 2 (alpha-amylase plus mucin)**

| | **V1** | **V2** | **V3** | **V4** | **V5** | V6 | **V7** | V8 |
|---|---|---|---|---|---|---|---|---|
| Dryness 4 | 0.452 a* | 0.555 a | 0.459 a | 0.473 a | 0.490 a | 0.382 a | 0.448 a | 0.471 a |
| Dryness 1 | 0.539 a | 0.835 a | 0.758 a | 0.528 a | 0.399 a | 0.296 a | 0.347 a | 0.302 a |
| Dryness 2 | 0.688 a | 0.869 a | 0.707 a | 0.496 a | 0.511 a | 0.352 a | 0.333 a | 0.290 a |
| Dryness 3 | 0.480 a | 0.560 a | 0.457 a | 0.395 a | 0.374 a | 0.298 a | 0.346 a | 0.339 a |
| Prob > F | 0.280 | 0.118 | 0.076 | 0.636 | 0.430 | 0.739 | 0.337 | 0.148 |
| Significance | No | No | No | No | No | No | No | No |

| | | | | | | | | |
|---|---|---|---|---|---|---|---|---|
| *Values followed by the same letter in a column are not different (P: 0.05) | | | | | | | | |

Contrary to what was obtained for Protein 1, the statistical analysis of variance and comparison by means of *Tukey's Test,* shows that the assay carried out with Protein 2 for model wine with the addition of skin tannins, only the displacement speeds of 2 and 3 mm/s are able to discriminate between samples of different levels of astringency, with the speed of 3 mm/s presenting the best results. On the other hand, for studies carried out using seed tannin, the results show that using artificial saliva with Protein 2 it is not possible to clearly differentiate samples of different levels of dryness.

In conclusion, with the development of these experiments, it was decided to carry out tribological measurements of the red wine samples preferably using artificial saliva with the addition of Protein 1 (alpha-amylase 1 mg/L) because this protein allows for discrimination by means of the friction coefficient, in a better way, samples of model wine of different levels of astringency and dryness.

### Example 3: Comparative results of astringency values evaluated with sensory panel and results obtained with the method of invention

In order to evaluate the coefficient of friction in red wine, wines were selected according to their levels of astringency (Table 6) to measure the coefficient of friction and analyze its relationship with the levels of astringency shown in a sensory evaluation.

**Table 6. Characterization of Cabernet Sauvignon wines.**

| | Astringent | Tannic Drying |
|---|---|---|
| 20088 | 2 | 2 |
| 20077 | 3 | 4 |
| 20109 | 3 | 6 |
| 20073 | 5 | 7 |
| 20078 | 8 | 7 |
| 20089 | 10 | 13 |
| 20092 | 12 | 15 |
| 20043 | 14 | 17 |

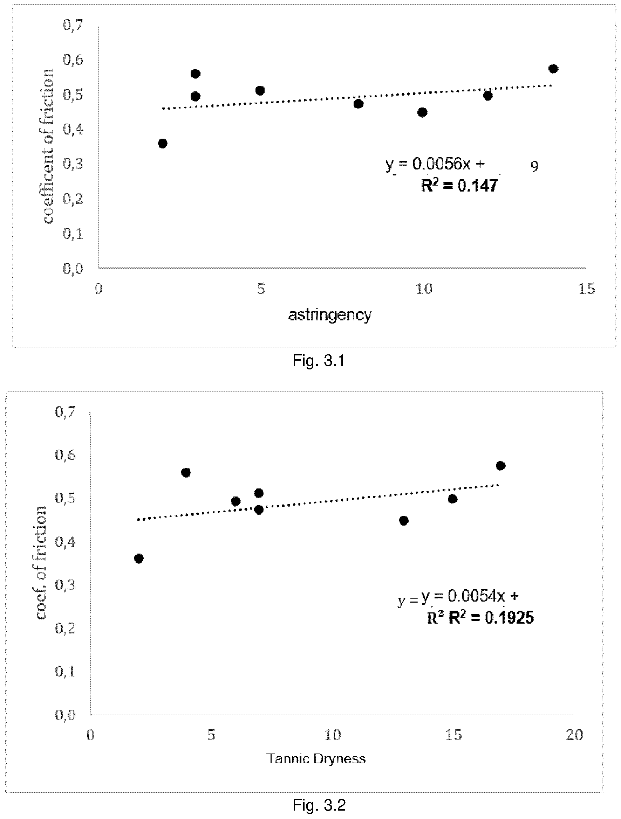

Figures 3.1 and 3.2 show the low interaction between the coefficient of friction and astringency and perceived dryness in red wines without mixing with artificial saliva.

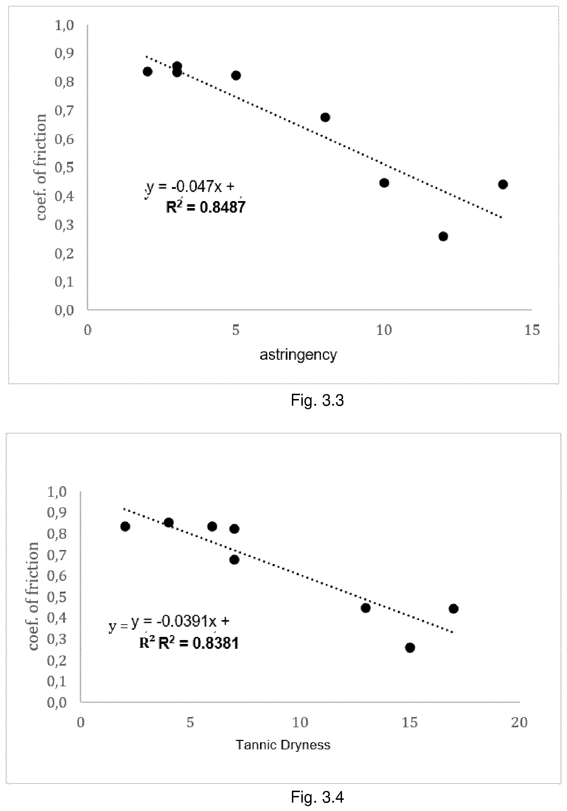

Figure 3.3 shows the relationship between the astringency perceived by a sensory panel and the coefficient of friction when red wine is mixed with artificial saliva, which decreases with increasing astringency. Regarding the dryness perceived by the sensory panel, the behavior is similar to what occured with astringency, the coefficient of friction decreases as the perceived dryness increases (Figure 3.4). It should be noted that this behavior in the coefficient of friction is only observed when the wine is combined with artificial saliva in the same proportion.

### Example 4: Kit

The MTM2 tribometer (Mini Traction Machine 2, PCS Instruments) is preferentially used in the condition of Sliding Rotation Ratio (SRR) of 50% and analysis of the coefficient of friction obtained under conditions of fixed speed at 5mm/S, 50%SRR and 1 Newton of load for 30 minutes.

The kit used consists of:
1. an artificial saliva-type protein composition consisting of sachets of 5 mg alpha-amylase for dilution in ultrapure water (Milli-Q or equivalent) or distilled water up to a recommended concentration of 1 mg/L;
2. tribological pair composed of polydimethylsiloxane disc (PDMS), PDMS ball both with hardness index 50 (HARD 50) and ball with a size of ¾"; and
3. instructions for use, including preparation and stirring time of the artificial saliva solution before use, proportion of artificial saliva and drink to be mixed, temperature conditions, travel speed, time and force for tribological analysis.

## Claims

1. A method for quantifying the astringency of a beverage **CHARACTERIZED in that** the method comprises the steps of
a) Contacting an artificial saliva-type protein composition with the beverage in a ratio between 0.75:2 and 1.75:0.5; and
b) Determining the tribological profile using a tribological device; and
c) Determining the astringency of the beverage;
where the protein composition contains alpha-amylase and optionally includes at least one protein selected from mucins, amylases, proline-rich proteins, and lysozymes;
where the concentration of protein in the protein composition is between 0.5 to 2.5 mg/L.

2. The method according to claim 1, **CHARACTERIZED in that** the beverage corresponds to beer, cider or wine.

3. The method according to claim 1, **CHARACTERIZED in that** the beverage corresponds to red wine.

4. The method according to claim 1, **CHARACTERIZED in that** the ratio of protein composition in mixture with the beverage is between 0.75:1.5 to 1.25:1.

5. The method according to claim 1, **CHARACTERIZED in that** the protein concentration in the protein composition is between 0.75 and 1.5 mg/L.

6. The method according to claim 5, **CHARACTERIZED in that** the protein concentration in the protein composition is 1 mg/L.

7. The method according to claim 1, **CHARACTERIZED in that** in step b) the coefficient of friction is analyzed by subjecting the sample to a normal force of 1 to 5 N and at a temperature between 25 and 30ºC.

8. The method according to claim 1, **CHARACTERIZED in that** step b) is performed by subjecting the sample to a normal force for between 10-40 min, preferably between 25 to 30 min.

9. An artificial saliva-type protein composition to quantify the astringency of a beverage **CHARACTERIZED in that** it comprises a protein mixture comprising of alpha-amylase and optionally including at least one protein selected from mucins, amylases, proline-rich proteins, and lysozymes, in ultrapure or distilled water.

10. The protein composition according to claim 9, **CHARACTERIZED in that** the protein concentration in the protein composition is between 0.5 and 2.5 mg/L.

11. A kit to quantify the astringency of a beverage **CHARACTERIZED in that** it comprises:
- an artificial saliva-type protein composition that contains alpha-amylase and optionally includes at least one protein selected from mucins, amylases, proline-rich proteins and lysozymes where the concentration of protein in the solution is between 0.75 and 1.5 mg/L;
- a tribological device comprising a tribological pair consisting of a polydimethylsiloxane disc (PDMS), a PDMS ball both of hardness index 30 or 50 (HARD 30 or HARD 50) and the ball of a size from 1/2" to 3/4".
